# EUROPEAN PATENT SPECIFICATION

(11) **EP 1 400 222 B1**
(45) Date of publication and mention of the grant of the patent: **03.01.2007**
(21) Application number: 03017672.1
(22) Date of filing: 14.08.2003
(51) Int. Cl.: A61F 5/445, A61F 5/44

(54) **Pouch for medical use**
Beutel für medizinischen Gebrauch
Poche à usage médical

(30) Priority: 23.09.2002 US 412951 P; 06.03.2003 US 452585 P
(43) Date of publication of application: 24.03.2004
(73) Proprietor: Bristol-Myers Squibb Company, New York, N.Y. 10154 (US)
(72) Inventor: Tsai, Lawrence M., Holmdel, NJ 07733 (US); Meytus, Sam, E. Brunswick, NJ 08816 (US); Corchado, Eligio, Union, NJ 07083 (US)
(74) Representative: Holmes, Miles Keeton

(56) References cited:
- EP-A- 0 259 184
- US-A- 4 790 051
- US-A- 4 816 027
- US-A- 5 647 670
- US-A- 5 690 622
- US-A- 5 865 819
- US-B1- 6 231 553

## Description

The present invention relates to a pouch for medical use. The term "medical" may include personal hygiene. In one form, the pouch may be a collection pouch (such as an ostomy pouch) for collecting body fluids. One aspect of the invention may relate to a medical pouch including separable inner and outer pouches. Another aspect of the invention may relate to a reclosable medical pouch.

### BACKGROUND TO THE INVENTION

Two pouch systems (including an inner and an outer pouch) have been suggested as an alternative to a single pouch ostomy system. However, there remains a need for a pouch design that can combine any of convenience of use, ease of disposal, ergonomic design and security and assurance against leaks, for good customer acceptance. There also remains a need for a reclosable pouch system for medical use, that can combine any of convenience of use, ease of use, ergonomic design, security and assurance against leaks.

Reference may be made to EP-A-0259184 which discloses the pre-characterizing features of the invention. Reference may also be made to US-A-6231553.

### SUMMARY OF THE INVENTION

The present invention is defined in the claims.

Advantages of the invention may include providing a pouch design that may meet one or more of the following, generally conflicting requirements: ease of use, ease of disposal of a waste-containing inner pouch; security and assurance against pouch leakage; and/or ease of manufacture.

### DESCRIPTION OF THE DRAWINGS

Non-limiting preferred embodiments of the invention are now described, by way of example, only, with reference to the accompanying drawings, in which:
Fig. 1 is a schematic section through a first pouch embodiment;
Fig. 2 is a schematic section similar to Fig. 1, but showing the outer pouch in an opened condition;
Fig. 3 is a schematic section through a second pouch embodiment;
Fig. 4 is a schematic section through a third pouch embodiment;
Fig. 5 is a schematic section through a zipper of the third embodiment;
Fig. 6 is a schematic section along the line VI-VI of Fig. 5;
Fig. 7 is a schematic section along the line VII-VII of Fig. 5;
Fig. 8 is a schematic view showing the outer pouch opened, and the inner pouch just prior to insertion in the outer pouch/just following removal from the outer pouch;
Fig. 9 is a schematic view similar to Fig. 8, with the outer pouch open, but showing the inner pouch fastened to the outer pouch; and
Figs. 10 is a schematic view similar to Fig. 9, but showing the outer pouch closed.

### DESCRIPTION OF PREFERRED EMBODIMENTS

The preferred embodiments present various examples of a pouch for medical use. In one form, the pouch is a collection pouch in the form of an ostomy pouch. The preferred embodiments illustrate a multi-pouch system. However, it will be appreciated that principles of the invention may be equally applied to a single pouch for medical use. Corresponding reference numerals may denote corresponding features, where appropriate.

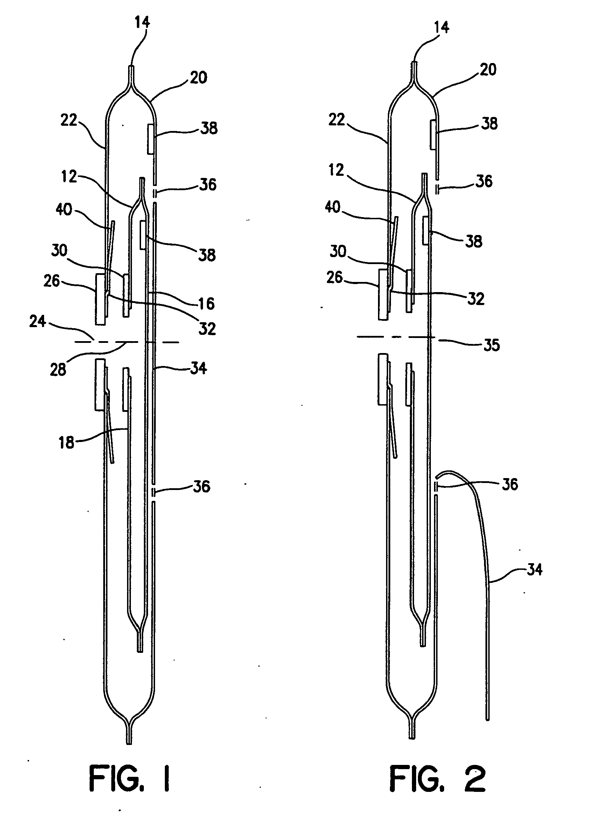

Referring to Fig. 1, an ostomy pouch 10 generally comprises an inner pouch 12 and an outer pouch 14. The inner pouch 12 may include a front wall 16 and a rear wall 18 secured together around their mutual periphery. The outer pouch 14 may include a front wall 20 and a rear wall 22 secured together around their mutual periphery. For each pouch 12 and 14, the front and rear walls may be formed of separate sheets secured together, or they may be formed from a common sheet folded at a periphery of the respective pouch 12 or 14.

The outer pouch 14 may include, in its rear wall 22, a first waste inlet aperture 24. An adhesive body fitment 26 may be coupled, fixedly or removably, to the outer pouch 14 around the stoma aperture 24. The body fitment 26 may include a wafer or pad of a skin-friendly adhesive for securing the ostomy pouch 10 to the peristomal region of a wearer. For example, the adhesive may be a hydrocolloid adhesive. In this embodiment, the body fitment 26 is fixedly secured to the rear wall 22 of the outer pouch 14, for example, by welding, heat sealing or adhesive, to be integral with the outer pouch 14.

The inner pouch 12 may include, in its rear wall 18, a second waste inlet aperture 28. The second waste inlet aperture 28 may be slightly bigger than the first waste inlet aperture 24. A first fastener 30, 32 may be provided for fastening the inner pouch 12 to the outer pouch 14 or to the body fitment 26. In the fastened position (not shown), the first and second inlet apertures 24 and 26 may be at least partly, or substantially, aligned to provide communication between an interior of the inner pouch 12, and the first inlet aperture. Body waste entering the pouch 10 passes directly into the inner pouch 12. The first fastener 30, 32 preferably provides a substantially liquid-tight and/or gas-tight seal, to prevent waste matter from escaping into the outer pouch 14.

The first fastener 30, 32 may be a releasable or separable fastener, to enable the inner pouch 12 to be separated from the outer pouch 14/body fitment 26. The first fastener 30, 32 may be a mechanical fastener, or an adhesive fastener. Generally, the first fastener may include a first coupling member 30 surrounding or bordering the second waste inlet aperture, for cooperating (mechanically or adhesively) with a second coupling member 32 carried by the outer pouch 14 or the body fitment 26. The coupling members 30 and 32 may be flat or plate-like, or they may have mechanical engagement profiles.

In this embodiment, an adhesive fastener may be preferred. For example, the first coupling member 30 may be an adhesive faceplate member 30 bordering or surrounding the second waste inlet aperture 28. The second coupling member 32 may be a non-adhesive receiving surface (or "landing" surface) 32 to which the adhesive member 30 may be attached. The adhesive may of a tacky or non-tacky type. A suitable material for the adhesive member 30 may be a resealable foam tape, such as the type manufactured by 3M company of Minneapolis, Minnesota, and designated 9776 Foam Medical Tape on Liner. The resealable foam tape 30 may include a closed cell polyethylene foam backing approximately 0.8 mm thick with a hypoallergenic pressure sensitive acrylate adhesive that faces towards the landing surface 32. Another suitable material is a sheet substrate with pressure sensitive adhesive instead of a foam. In either case, the adhesive may initially be protected by a silicone release paper (not shown) prior to first use of the pouch. The landing surface 32 may be provide by a flexible film of plastics, for example, thermoplastics.

Referring to both Figs. 1 and 2, a wall, for example, the front wall 20, of the outer pouch 14 may be partly, or substantially entirely, openable to provide access for fitting or removing the inner pouch 12. In this embodiment, an openable portion 34 of the front wall 20 may be provided, in the form of a door, flap or hatch portion, to define an access window 35 in the front wall 20. The access window 35 may be of approximately the same size as the inner pouch 12, or it may be larger or smaller than the inner pouch. Generally, a larger size may aid easier insertion and removal of the inner pouch 12. However, the inner pouch 12 is flexible, and may be quite easily inserted or removed through a smaller-size access window 35. The access window 35 may be larger than the outer periphery of the first coupling member 30 of the inner pouch 12.
A second fastener 36 may be provided for fastening closed the openable portion 34. The second fastener 36 preferably provides a liquid-tight and/or gas-tight seal. The second fastener 36 may extend around, or border, a periphery of the openable portion 34 and/or the corresponding confronting edge of the access window 35 in the front wall 20. The second fastener 36 may, for example, be a zip fastener, a sliding zip fastener, a hook-loop fastener (e.g., Velcro ™), a hook-hook (e.g., male-male interlocking) fastener, or a repositionable adhesive fastener (similar to that described for the first fastener 30, 32), or a magnet fastener.

A deodorising filter 38 may be fitted to one or both of the inner pouch 12 and the outer pouch 14 for deodorising vented flatus. The deodorising filter 38 may be fitted to an interior facing surface of the respective pouch (as in the drawings), or to an exterior facing surface of the respective pouch 12, 14. The deodorising filter 38 may be fitted to communicate with a gas vent aperture (not shown) in the respective pouch wall.

Referring to Fig. 2, in use, in order to insert the inner pouch 12 into the outer pouch 14, the second fastener 36 is unfastened to release the openable portion 34 of the front wall 20. The inner pouch 12 may then be inserted through the open access window 35 in the front wall 20, and pressed into fastening engagement with, for example, the body fitment 26. In the case of the adhesive member 30 described above, the adhesive member 30 is pressed into adhesive engagement with the landing surface 32 of the body-fitment 26. Thereafter, the openable portion 34 is closed and the second fastener 36 is refastened (Fig. 1) .

Referring again to Fig. 2, in order to remove the inner pouch 12, for example when the inner pouch 12 has become full, the second fastener 36 is unfastened to release the openable portion 34 of the front wall 20. The ostomate may then fold back a portion of the inner pouch 12 (not shown), to gain access to the first fastener 30, 32. For example, by folding down the upper portion of the inner pouch, the ostomate may gain access to the upper edge of the first fastener 30, 32. The inner pouch 12 may then be separated from the body fitment 26, for example, by peeling the first coupling member 30 free of the second coupling member 32. As can be seen in both Figs. 1 and 2, the second coupling member 32 may be larger than the first coupling member 30 (or may include one or more extension tabs) to provide a gripping surface or portion 40 that the ostomate can grip to counterbalance the peeling forces, and to at least partly isolate the peeling forces from the body fitment 26. Once the inner pouch 12 has been separated, the inner pouch 12 can be withdrawn from the outer pouch 14 through the access window 35.

The inner pouch 12 may be inserted and/or removed while the outer pouch 14 is being worn on the body. Alternatively, the inner pouch 12 may be inserted and/or removed while the outer pouch 14 is not being worn.

The inner pouch 12 may be intended to be disposed of in a flushable toilet. The inner pouch may be of material that is able to withstand body waste and/or other wet contents during normal usage, but is able to disintegrate or at least soften to facilitate disposal in a flushable toilet. The material may be "activateable" by addition of an activating agent to the toilet water, or to the interior or exterior of the inner pouch 12. For example, the additive may be in the form of a tablet, or powder, or liquid that may be added to the toilet water. Alternatively, the additive may be in the form of a liquid, an aerosol, or cream that can be sprayed or distributed onto the surface of the inner pouch 12 using a spray or other applicator.

In one example, the inner pouch 12 may be made partly, or entirely, of a pH-sensitive polymer that becomes soluble or dispersible in either or both of acid or alkaline conditions (for example, pH below about 4 and/or pH greater than about 10). The activating agent may be an acid or alkaline additive. Example alkaline compounds may include common base chemicals, such as sodium carbonate, sodium metasilicate, sodium hydroxide, trisodium phosphate, metal mixture compounds, etc.

Another means to soften a polymer material of the inner pouch 12 is to use an organic solvent as the activating agent. For example, the polymer material may be a pH-sensitive polymer. At least some pH-sensitive polymers may be based on acrylic acid or acrylate, and may be dissolved in certain solvents relatively quickly, such as acetone, isopropyl alcohol, amino propanol, ethanol amine, etc.

Another possibility may be for the inner pouch 12 to be made partly, or entirely, of a biodegradable polymer that is water resistant (to withstand the waste contents). The pouch material may be "activated", for example, by spraying a chemical onto the inner pouch 12, to activate the disintegration for the pouch to become flushable or dispersible in a toilet.

Another possibility may be for the inner pouch 12 to be made partly, or entirely, of a laminate including a water-soluble or water-dispersible polymer as a bulk layer, and a thin layer of a water-resistant coating applied to a surface. For example, the water-resistant coating may be applied to the interior facing surface of the inner pouch 12, in order to prevent, or at least obstruct, moisture from the collected waste from damaging the integrity of the material. The exterior surface of the inner pouch 12 may, in normal use of the inner pouch, be protected by the outer pouch 14. However, when the inner pouch 12 is removed from the outer pouch 14 and placed in a toilet, the bulk layer of the laminate may be exposed to the toilet water, and may disintegrate or disperse relatively quickly. The remaining water-resistant coating of the inner pouch 12 may break down physically, as the layer may be too thin to support the weight of the body waste content, or to withstand water agitation during flushing. This technique may not involve any activation agent to activate the material.

Another possibility is a V-shape pouch, with or without the use of a carrier sleeve, such that the pouch can flow in streamlined fashion through the passages of a toilet and any sewer or septic line connected to the toilets.

The inner pouch 12 may be made of, or include, a barrier material to prevent odours from transpiring through the material of the pouch wall 12. If a deodorising filter 38 is provided on the inner pouch 12, then the deodorising filter may be sufficiently small that it can be flushed easily in a toilet system without causing blockage. A deodorising filter 38 may be used on the inner pouch 12 even when the inner pouch 12 is not made of, or does not include, a barrier material. A deodorising agent may also or alternatively be coated onto the material of the inner pouch 12 to deodorise any flatus transpiring through the wall material of the inner pouch 12.

The first coupling member 30 may be removable from the inner pouch 12 prior to disposal, or may itself be made of a material that disintegrates or disperses in the toilet, or may be configured (e.g. sufficiently small) that it does not significantly obstruct flushing and/or can itself be flushed away easily.

The outer pouch 14 may be intended to be used multiple times with replacement inner pouches 12. For each replacement of the inner pouch 12, the outer pouch 14 may remain worn on the body or may be removed and then refitted to the body. The outer pouch 14 may be configured to provide protection for the inner pouch 12. The inner pouch 12 may be more fragile than the outer pouch 14 to facilitate at least partial disintegration of the inner pouch 12 in a toilet. The outer pouch 14 may also provide additional security to collect any waste matter that may accidentally escape or leak from the inner pouch 12.

The outer pouch 14 may made of one or more conventional pouch materials not optimised for disposal in a flushable toilet. The outer pouch 14 may be made of a coextruded film containing heat sealable material as outer skins, and an odour barrier material in between. For example, the heat sealable material may be Polyethylene (PE), or polypropylene (PP), polybutene (PB), or ethylene vinyl acetate (EVA), or ethylene methyl acrylate acetate (EMA), or ethylene acrylic acid (EAA), etc. The odour barrier material may, for example, be polyvinylidene chloride (PVDC), or ethylene vinyl alcohol (EVOH), or nylons, etc. An example film for the outer pouch 14 may be MF Film ™ made by Sealed Air Cryovac, or Saranex Film ™ made by Dow Chemicals.

The front wall 20 and/or rear wall 22 of the outer pouch 14 may also include a comfort layer on the exterior surface, to provide a soft, comfortable cloth-like surface. The comfort layer may, for example, be of a non-woven material.

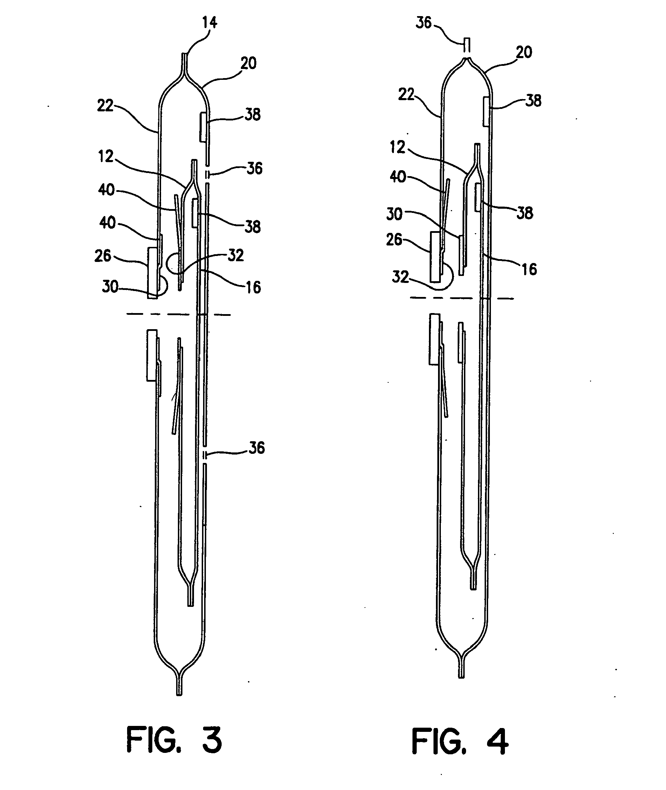

Fig. 3 illustrates a second embodiment very similar to the first embodiment. The main difference in the second embodiment is that positions of the first and second coupling members 30 and 32 are swapped. The first coupling member (e.g., adhesive member) 30 is mounted on the body fitment 26, and the second coupling member (e.g., landing surface) 32 is mounted on the inner pouch 12. Arranging the first and second coupling members 30 and 32 in this way may avoid disposal of the adhesive member 30 in the toilet. Instead, the landing surface 32 may be intended for disposal in the toilet as part of the inner pouch 12. The landing surface may be made of the same activatable and/or disintegratable and/or dispersible material as the inner pouch 12. The example resealable adhesive referred to in the first embodiment may permit repeated adhesion of the same adhesive member 30, thereby allowing multiple replacement inner pouches 12 to be adhered, in turn, to the same outer pouch 14 and body fitment 26.

Figs. 4-10 illustrate a third embodiment very similar to the first embodiment. The main difference in the third embodiment is that the second fastener 36 is integrated in the seam joining the front wall 20 and rear wall 22 of the outer pouch 14. The openable portion 34 of the front wall 20 may therefore be the entire width of the outer pouch 14, providing a wide access window 35. A fixed seam of the outer pouch 14 therefore may not extend around the entire periphery of the outer pouch 14. Instead the second fastener 36 may extend partly or entirely around the periphery. The second fastener 36 may be any of the fastener types referred to in the first embodiment, but a sliding zipper is preferred and is now described further.

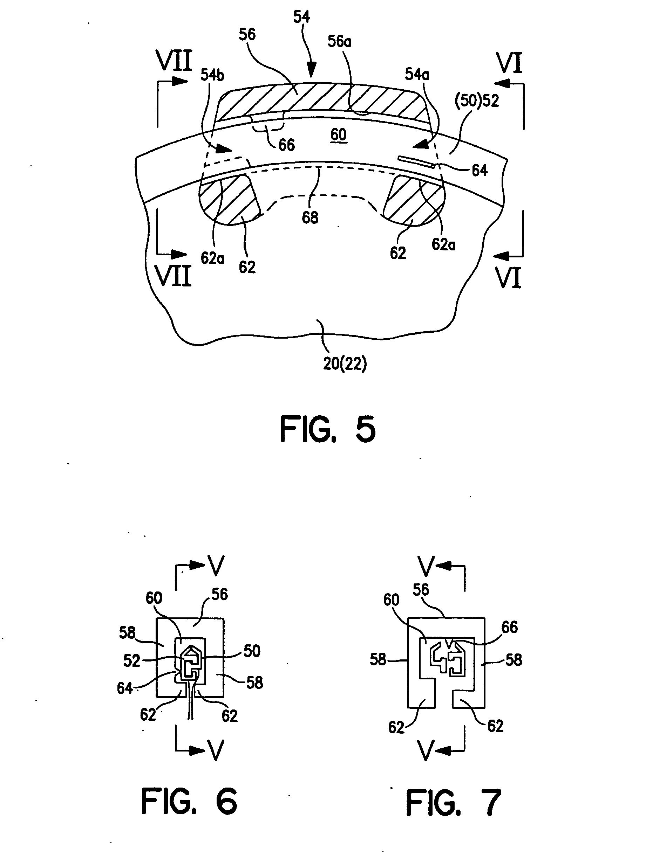

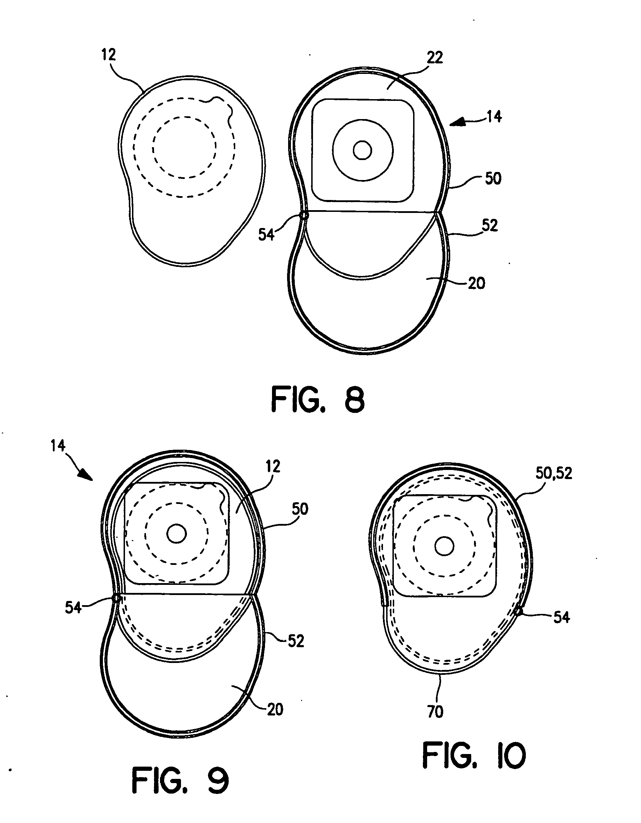

Referring to Figs. 5-7, a first zipper track 50 may be attached to one of the front and rear walls 20, 22 of the outer pouch 14 in Fig 4 (alternatively, the attachment may be to 20, 34 in the embodiments of Figs. 1 - 3 as the flap opening is on the face instead of the seam), and a second zipper track 52 may be attached to the other of the front and rear walls 20, 22 of the outer pouch 14 in Fig. 4. The first and second zipper tracks 50, 52 may be male and female tracks, respectively. The zipper tracks 50 52 may be generally curved to match the curved peripheral shape of the outer pouch 14 (for example, as seen in Figs. 8-10). A zipper slider 54 may be captive on the tracks 50 and 52 for fastening and unfastening the zip tracks 50, 52. The slider 54 may be generally channel shaped, and include a bridge 56 and two depending side walls 58 defining a channel region 60. The side walls 58 of the slider 54 may carry guides 62 that engage behind the tracks 50, 52 to hold the slider 54 captive on the tracks. The channel region 60 may be narrower at one end 54a (e.g. the right as seen in Fig. 5) of the slider 54 than the other end 54b. Moving the slider 54, for example, leftwards fastens the zip by pressing the male and female tracks 50, 52 into mutual engagement with each other. A pressing projection 64 located near the end 54a of the slider 54 may aid application of pressure to urge the tracks 50, 52 together. Moving the slider 54, for example, rightwards unfastens the zip by separating the male and female tracks 50, 52. Separation may be aided by a separation projection (blade) 66 depending from the bridge 56 and/or by a difference in the height of the guides 62 at the end 54b of the slider to promote lifting of one track relative to the other.

As best seen in Fig. 5, the slider 54 may have a curved characteristic that matches and/or accommodates the curved shape of the zipper tracks 50, 52. The curved characteristic may define a curvature that is not significantly smaller (e.g., radius of curvature is not significantly greater) than the maximum curvature (e.g., minimum radius of curvature) of the periphery of the outer pouch 14 over which the zip extends. For example, the curved characteristic may be an interior surface 56a of the bridge 56, which has a curvature that is not significantly smaller than the maximum curvature of upper periphery of the pouch. (The bridge surface 56a may be shaped to have a radius of curvature that is not significantly greater than the minimum radius of curvature of the upper periphery of the pouch). The bride 56 may act as a guide for guiding movement of the slider 54 relative to the tracks 50, 52. Additionally or alternatively, the curved characteristic may be defined by surfaces 62a of the guides 62 that confront the zipper tracks 50, 52. The guide surfaces 62a may be inclined or curved along an arc 68 having a curvature that is not significantly smaller than the maximum curvature of the upper periphery of the pouch. (The arc 68 may have a radius of curvature that is not significantly greater than the minimum radius of curvature of the upper periphery of the pouch).

Such a curved characteristic may enable the slider 54 to slide smoothly around the curved shape of the outer pouch 14. This may be especially advantageous for the elderly or less dextrous, or for ostomates with sensitive stomas who wish to avoid discomfort as the slider 54 is operated.

Referring to Figs. 8-10, the second fastener (zipper) 36 may extend for a half, or more, of the periphery of the outer pouch 14. In the form illustrated, the length of the zipper 36 may be about two-thirds, or more, of the periphery. Such a length of zipper 36 allows the inner pouch 12 easily to be inserted in, or removed from, the outer pouch 14, and it may also provide substantially unhindered access to the first fastener (30, 32) for removably securing the inner pouch 12 in position.

The zipper 36 may be arranged to open an upper portion of the outer pouch 14. Opening an upper portion may be advantageous in providing access to the stoma aperture. A lower portion of the outer pouch 14 may have a permanently sealed seam 70. The permanently sealed seam 70 may act as a failsafe to ensure that the inner pouch 12 cannot accidentally drop downwardly out of the outer pouch 14.

The zipper may be made of any suitable material, such as metal or plastics or fabrics.

The invention, particularly as described in the preferred embodiments, can provide a pouch design that may meet multiple, generally conflicting requirements, in terms of ease of use, ease of disposal of a waste-containing inner pouch, security and assurance of the wearer against pouch leakage, and ease of manufacture.

## Claims

1. An ostomy pouch comprising:
an inner pouch (12);
an outer pouch (14) having front (20) and rear (22) walls; and
a body fitment (26) for securing the ostomy pouch to a wearer, the body fitment being attached to the rear wall of the outer pouch for supporting at least the outer pouch;
**characterized in that**:
the front wall (20) of the outer pouch includes a portion (34; 20) that is openable and allows replacement of the inner pouch while the rear wall of the outer pouch remains intact with the body fitment; and
the outer pouch further comprises a fastener (36) configured to permit reclosable opening of said openable portion of the front wall of the outer pouch.

2. A pouch according to claim 1, wherein the fastener (36) is a sliding zipper fastener.

3. A pouch according to claim 1 or 2, wherein the openable portion of the front wall of the outer pouch comprises a flap portion (34), and the fastener (36) reclosably fastens said flap portion and an adjacent portion of the front wall.

4. A pouch according to claim 1 and 2, wherein the fastener (36) extends along a portion of a peripheral seam between the front and rear walls (20, 22).

5. A pouch according to any preceding claim, wherein the outer pouch (14) comprises a first aperture (24), and the inner pouch (12) comprises a second aperture (28), and wherein at least in an operative position of the inner pouch within the outer pouch, the first and second apertures (24, 28) are at least partly aligned for communication.

6. A pouch according to any preceding claim, further comprising a separable coupling (30, 32) for removably securing the inner pouch (12) directly or indirectly to the outer pouch (14), the separable coupling permitting replacement of the inner pouch by a replacement inner pouch.

7. A pouch according to claim 6, wherein the separable coupling (30, 32) removably secures the inner pouch to the outer pouch or to the body fitment (26).

8. A pouch according to claim 6 or 7, wherein the separable coupling (30, 32) is an adhesive coupling.

9. A pouch according to any preceding claim, wherein the inner pouch (12) being is configured for (i) removal from the outer pouch (14), and (ii) disposal in a flushable water closet.

10. A pouch according to claim 9, wherein inner pouch (12) comprises water disintegrateable or water dispersible material.

11. A pouch according to claim 2 or any claim dependent thereon, wherein the zipper (36) extends along a path including a curved portion.

12. A pouch according to claim 11, wherein the path is predominantly curved.

13. A pouch according to claim 11 or 12, wherein the sliding zipper comprises at least one zipper track (50, 52) and a movable slider (54), the slider comprising one or more track engaging surfaces (56a, 62a) having a characteristic to accommodate a curvature of the path of the slider along the track.

## Patentansprüche

1. Ostomiebeutel, der umfasst:
einen inneren Beutel (12);
einen äußeren Beutel (14) mit Vorderwand (20) und Rückwand (22); und
ein Körperanbringungselement (26) zum Befestigen des Ostomiebeutels an einem Träger, wobei das Körperanbringungselement an der Rückwand des äußeren Beutels angebracht ist, um mindestens den äußeren Beutel zu stützen;
**dadurch gekennzeichnet dass**:
die Vorderwand (20) des äußeren Beutels einen Abschnitt (34; 20) aufweist, der zu öffnen ist und das Ersetzen des inneren Beutels gestattet, während die Rückwand des äußeren Beutels mit dem Körperanbringungselement intakt bleibt; und
der äußere Beutel weiterhin ein Verbindungselement (36) aufweist, das so konfiguriert ist, dass es das wiederverschließbare Öffnen des zu öffnenden Abschnitts der Vorderwand des äußeren Beutels gestattet.

2. Beutel nach Anspruch 1, wobei das Verbindungselement (36) ein Schiebezippverschluss-Verbindungselement ist.

3. Beutel nach Anspruch 1 oder 2, wobei der zu öffnende Abschnitt der Vorderwand des äußeren Beutels einen Klappenabschnitt (34) aufweist und das Verbindungselement (36) den Klappenabschnitt und einen angrenzenden Abschnitt der Vorderwand wiederverschließbar verbindet.

4. Beutel nach Anspruch 1 und 2, wobei das Verbindungselement (36) sich entlang einem Abschnitt einer peripheren Naht zwischen der Vorder- und der Rückwand (20, 22) erstreckt.

5. Beutel nach einem der vorhergehenden Ansprüche, wobei der äußere Beutel (14) eine erste Öffnung (24) aufweist und der innere Beutel (12) eine zweite Öffnung (28) aufweist, und wobei mindestens in einer Einsatzstellung des inneren Beutels innerhalb des äußeren Beutels, die erste und die zweite Öffnung (24, 28) mindestens teilweise zwecks Verbindung fluchten.

6. Beutel nach einem der vorhergehenden Ansprüche, der weiterhin eine trennbare Kupplung (30, 32) zum abnehmbaren Befestigen des inneren Beutels (12) direkt oder indirekt am äußeren Beutel (14) aufweist, wobei die trennbare Kupplung den Ersatz des inneren Beutels durch einen inneren Ersatzbeutel gestattet.

7. Beutel nach Anspruch 6, wobei die trennbare Kupplung (30, 32) den inneren Beutel abnehmbar am äußeren Beutel oder am Körperanbringungselement (26) befestigt.

8. Beutel nach Anspruch 6 oder 7, wobei die trennbare Kupplung (30, 32) eine klebende Kupplung ist.

9. Beutel nach einem der vorhergehenden Ansprüche, wobei der innere Beutel (12) zum (i) Entfernen aus dem äußeren Beutel (14) und (ii) zum Entsorgen in eine spülbare Wassertoilette konfiguriert ist.

10. Beutel nach Anspruch 9, wobei der innere Beutel (12) wasserauflösbares oder wasserdispergierbares Material umfasst.

11. Beutel nach Anspruch 2 oder einem davon abhängigen Anspruch, wobei sich der Zippverschluss (36) entlang einem Weg, der einen gekrümmten Abschnitt aufweist, erstreckt.

12. Beutel nach Anspruch 11, wobei der Weg überwiegend gekrümmt ist.

13. Beutel nach Anspruch 11 oder 12, wobei der Schiebezippverschluss mindestens eine Zippverschlussbahn (50, 52) und einen beweglichen Schieber (54) umfasst, wobei der Schieber eine oder mehrere mit der Bahn in Eingriff befindliche Oberflächen (56a, 62a) aufweist, die ein Merkmal, eine Krümmung des Weges des Schiebers entlang der Bahn auszugleichen, aufweisen.

## Revendications

1. Poche de stomie comprenant :
une poche intérieure (12),
une poche extérieure (14) ayant des parois avant (20) et arrière (22), et
un élément d'ajustement au corps (26) destiné à fixer la poche de stomie à un porteur, l'élément d'ajustement au corps étant fixé à la paroi arrière de la poche extérieure afin de supporter au moins la poche extérieure,
**caractérisée en ce que** :
la paroi avant (20) de la poche extérieure comprend une partie (34;20) qui peut s'ouvrir et qui permet le remplacement de la poche intérieure alors que la paroi arrière de la poche extérieure reste intacte avec l'ajustement au corps, et
la poche extérieure comprend en outre un dispositif de fixation (36) configuré pour permettre une ouverture refermable de ladite partie pouvant s'ouvrir de la paroi avant de la poche extérieure.

2. Poche selon la revendication 1, dans laquelle le dispositif de fixation (36) est un dispositif de fixation à glissière.

3. Poche selon la revendication 1 ou 2, dans laquelle la partie pouvant s'ouvrir de la paroi avant de la poche extérieure comprend une partie de rabat (34), et le dispositif de fixation (36) attache avec possibilité de refermer ladite partie de rabat et une partie adjacente de la paroi avant.

4. Poche selon la revendication 1 ou 2, dans laquelle le dispositif de fixation (36) s'étend le long d'une partie d'un raccord périphérique entre les parois avant et arrière (20,22).

5. Poche selon l'une quelconque des revendications précédentes, dans laquelle la poche extérieure (14) comprend une première ouverture (24), et la poche intérieure (12) comprend une seconde ouverture (28), et où au moins dans une position fonctionnelle de la poche intérieure à l'intérieur de la poche extérieure, les première et seconde ouvertures (24,28) sont au moins partiellement alignées en vue d'une communication.

6. Poche selon l'une quelconque des revendications précédentes, comprenant en outre un accouplement séparable (30,32) destiné à fixer de façon amovible la poche intérieure (12) directement ou indirectement à la poche extérieure (14), l'accouplement amovible permettant le remplacement de la poche intérieure par une poche intérieure de remplacement.

7. Poche selon la revendication 6, dans laquelle l'accouplement amovible (30,32), fixe de façon amovible la poche intérieure à la poche extérieure ou à l'élément d'ajustement au corps (26).

8. Poche selon la revendication 6 ou 7, dans laquelle l'accouplement amovible (30,32) est un accouplement adhésif.

9. Poche selon l'une quelconque des revendications précédentes, dans laquelle la poche intérieure (12) est configurée pour (i) le retrait de la poche extérieure (14), et (ii) le rejet dans des toilettes à chasse.

10. Poche selon la revendication 9, dans laquelle la poche intérieure (12) comprend un matériau dispersible dans l'eau ou pouvant se désintégrer dans l'eau.

11. Poche selon la revendication 2, ou une revendication quelconque dépendant de celle-ci, dans laquelle la fermeture à glissière (36) s'étend le long d'une voie comprenant une partie incurvée.

12. Poche selon la revendication 11, dans laquelle la voie est incurvée de façon prédominante.

13. Poche selon la revendication 11 ou 12, dans laquelle la fermeture à glissière comprend au moins une piste de fermeture à glissière (50,52) et un élément coulissant mobile (54), l'élément coulissant comprenant une ou plusieurs surfaces d'engagement de piste (56a, 62a) ayant pour caractéristique de s'adapter à la courbure de la voie de l'élément coulissant le long de la piste.
